# EUROPEAN PATENT APPLICATION

(11) **EP 4 328 238 A1**
(43) Date of publication of application: **28.02.2024**
(21) Application number: 22192040.8
(22) Date of filing: 24.08.2022
(51) Int. Cl.: C07K 14/435

(54) **PROTEINS FOR USE IN THE TREATMENT OF COMPLEMENT DYSREGULATION DISORDERS**

(71) Applicant: Ustav organicke chemie a biochemie AV CR, v.v.i., 166 10 Praha 6 (CZ)
(72) Inventor: Zoll, Sebastian, Praha (CZ); Sülzen, Hagen, Praha (CZ)
(74) Representative: Harber IP s.r.o.

(57) **Abstract**

The present invention relates to a protein derived from the extracellular domain of the surface protein ISG65 (invariant surface glycoprotein 65) of the human parasite *Trypanosoma brucei gambiense.* The ISG65-derived protein is useful in the treatment of complement dysregulation diseases.

## Description

### Field of Art

The present invention relates to proteins for use in the treatment of complement dysregulation disorders.

### Background Art

The complement cascade is a part of the innate immune system which complements the ability of antibodies and phagocytes to clear microbes and damaged cells and promote inflammation. The complement cascade is activated by three biochemical pathways: the classical pathway, the alternative pathway and the lectin pathway. The alternative pathway is responsible for the majority of terminal pathway activation. The alternative pathway is continuously activated at a low level, due to spontaneous hydrolysis of mildly unstable C3, generating C3b. C3b attaches to cell surfaces where it forms the nidus for formation of the C3 and C5 concertases which eventually lead to assembly of the membrane attack complex in the terminal pathway.

Excessive activation of complement proteins is often discovered to be the reason for many diseases. These include e.g. autoimmune diseases, Alzheimer's syndrome, schizophrenia, atypical hemolytic-uremic syndrome, angioedema, macular degeneration, and Crohn's disease (Tichaczek-Goska D.: Adv Clin Exp Med 2012 Jan-Feb;21(1): 105-14). Atypical hemolytic uremic syndrome (aHUS), C3 glomerulopathy (C3G), and paroxysmal nocturnal hemoglobinuria (PNH) are prototypical disorders of complement dysregulation (Wong E.K.S. and Kavangh D.: Semin Immunopathol 2018 Jan;40(1):49-64. doi: 10.1007/s00281-017-0663-8).

Mutations in the thioester domain (TED) of human C3b or in the C3b binding domain of human factor H, a negative regulator of the complement cascade, result in a loss of cellular self-recognition, uncontrolled lysis of red blood cells and consequently kidney failure. Treatment of these diseases currently requires, Eculizumab, a monoclonal antibody which targets C5. Due to the prohibitive cost of this drug cheaper alternatives are much needed.

### Summary of the Invention

The present invention is based on the finding that the extracellular domain of the surface protein ISG65 (invariant surface glycoprotein 65) of the human parasite *Trypanosoma brucei gambiense* is a complement receptor which targets the complement cascade at several critical intervention points. The extracellular domain of ISG65 binds to native C3, to its reactive activation products C3b and C3(H₂O), and to C3d. Addition of the extracellular domain of ISG65 to human serum resulted in a concentration-dependent decrease in haemolysis, indicating an inhibition of the alternative pathway. C3b serves as a central hub in the complement cascade and therefore its inhibition displays a more comprehensive approach for treatment of complement dysregulations than is targeting several downstream effector pathways (e.g. C5 or C9 polymerization). Also, unlike monoclonal antibodies, the ISG65 extracellular domain targets several interaction partners within the cascade, which potentially increases its potency.

The present invention thus provides an ISG65-derived protein for use as a medicament, in particular for use in the treatment of complement dysregulation diseases. "Dyregulation" herein refers in particular to excessive activation.

More specifically, the invention provides the ISG65-derived protein for use in the treatment of a disease selected from atypical hemolytic uremic syndrome (aHUS), C3 glomerulopathy (C3G), paroxysmal nocturnal hemoglobinuria (PNH), autoimmune diseases, Alzheimer's syndrome, schizophrenia, angioedema, macular degeneration, and Crohn's disease.

Native extracellular domain of ISG65 has the amino acid sequence SEQ ID NO: 1:

MMKYLLVFAIIATRIPVLLVIGSEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEVIAAAKEAGKKGAKIIIPAAAPATPTNSTKNDDSAPTEHVDRGIATNETQVEV
GIDADFDSLLDATEAAEVTRRHQRTAM.

The extracellular domain of ISG65 consists mainly of an approx. 80 Å (8 nm) long 3-helix bundle. Extensive loop structures stabilised by disulphide bonds establish the head domain which is located near the N-terminus of the protein and membrane-distal in the mature, membrane-embedded protein. The membrane-proximal parts of bundle helices 1 and 3 contain the majority of C3-TED interacting residues. Another interaction site is located in a loop connecting helices 2 and 3, just C-terminal of a short 3₁₀-helix.

The three interaction sites are underlined in SEQ ID NO. 1, and are the following:
site 1: 70-YIEFELYRIDYWLEKLNGPKGRKDGYAK-97 (SEQ ID NO: 2)
site 2: 210-DWSA-213 (SEQ ID NO: 3)
site 3: 290-NTAYAVNTKVEQE-302 (SEQ ID NO: 4)

Within the interaction sites, the identified crucial interface residues are: Tyr70, Phe73, Arg77, Trp81, Lys97; Trp211; Asn290, Tyr293, Thr297, Glu302.

The sequence of the extracellular domain of ISG65 used in the experiments presented in this patent application was SEQ ID NO: 5:

LLVIGSEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEVIAAAKEAGKKGAKIIIPAAAPATPTNSTKNDDSAPTEHVDRGIATNETQVEV
GID (SEQ ID NO: 5).

The longest, truncated sequence of the extracellular domain of ISG65 that can be produced recombinantly is SEQ ID NO: 6:

MMKYLLVFAIIATRIPVLLVIGSEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEVIAAAKEAGKKGAKIIIPAAAPATPTNSTKNDDSAPTEHVDRGIATNETQVEV
GIDAD (SEQ ID NO: 6).

In some embodiments, a sequence of the extracellular domain of ISG65 that can be produced recombinantly is SEQ ID NO: 7:

SEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEV (SEQ ID NO: 7).

Engineered variants of the native ISG65 extracellular domain sequence can be used, e.g. variants having a higher thermostability, lyophilizable variants, etc., or suitable isoforms (several native isoforms of ISG65 are known to date). These variants and isoforms are herein included in the term "ISG65-derived protein".

The term "ISG65-derived protein" herein refers to proteins having the length of up to 500 amino acid residues and containing an amino acid sequence having at least 90% identity, preferably at least 95% identity, to the sequence SEQ ID NO: 8, wherein the underlined regions are conserved:

GYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QE (SEQ ID NO: 8).

More preferably, the ISG65-derived protein is a protein having the length of up to 500 amino acid residues and containing an amino acid sequence having at least 90% identity, preferably at least 95% identity, to the sequence SEQ ID NO: 4, wherein the underlined regions are conserved.

In some embodiments, the ISG65-derived protein is a protein having the length of up to 500 amino acid residues and containing an amino acid sequence having at least 90% identity, preferably at least 95% identity, to the sequence SEQ ID NO: 5, wherein the underlined regions are conserved.

In some embodiments, the ISG65-derived protein is a protein having the length of up to 500 amino acid residues and containing an amino acid sequence having at least 90% identity, preferably at least 95% identity, to the sequence SEQ ID NO: 6, wherein the underlined regions are conserved.

The length of the ISG65-derived protein is up to 500 amino acid residues, more preferably up to 450 amino acid residues, in some embodiments up to 400 amino acid residues. The protein may be extended on one or both termini of the herein shown sequence. The extension sequence may contain, for example, an affinity tag, or a sequence with at least 90% identity to the corresponding ISG65 region.

Affinity tags are known in the art, and include, for example: ALFA-tag, AviTag, C-tag, polyglutamare tag, polyarginine tag, E-tag, FLAG-tag, HA-tag, His-tag, Myc-tag, NE-tag, Rho1D4-tag, S-tag, Softag1, Softag3, Spot-tag, Strep-tag, T7-tag, TC tag, Ty tag, V5 tag, VSV-tag, Xpress tag, Isopeptag, SpyTag, SnoopTag, DogTag, or SdyTag.

The term "identity" refers to the number of identical residues over the total length of the reference sequence. The reference alighment is the CLUSTAL 0(1.2.4) multiple sequence alignment (https://www.ebi.ac.uk/Tools/msa/clustalo/).

The percentage of identity lower than 100% refers to sequences differing from the reference sequence by terminal extensions, terminal deletions, point mutations, insertions and deletions within the sequence.

The present invention further provides a pharmaceutical formulation containing the ISG65-derived protein and at least one pharmaceutically acceptable excipient. The excipients may include solvents, such as water or a buffer, stabilizers, surfactants, binders, fillers and glidants. The formulations may be liquid or solid formulations. In solid formulations, the protein may preferably be lyophilized.

### Brief Description of Drawings

Fig. 1: Chromatogram showing purification of the ISG65:C3b complex (~220 kDa) using size exclusion chromatography. Complex formation of ISG65 with C3b was confirmed by analysing fractions underneath the main peak (marked with horizontal bar) on an SDS gel.
Fig. 2: Haemolysis assay measuring the lysis of red blood cells in human serum in presence of ISG65 and ISG75 (n =3; ± std S. D; ^{∗∗} indicates p ≤ 0.01; ^{∗∗∗}indicates p ≤ 0.001).
Fig. 3: Surface plasmon resonance sensorgrams showing the kinetics of ISG65 binding to C3d, C3(H₂O), C3b, C3, and C3c. Biotinylated ISG65 (ligand) was immobilised, and different concentrations of indicated analytes were allowed to flow over ISG65.
Fig. 4: Cryo-EM structures of ISG65 in complex with C3 and C3b. A, Cryo-EM density maps showing side views of ISG65:C3 (left), and ISG65:C3b (right) complexes at 2 different angles. C3(b) domains are labelled. B, Cartoon representation of ISG65:C3 and ISG65:C3b. Interacting domains are indicated in black.
Fig. 5: ISG65 binding to C3 ANA/C3a. A, Close-up view of the interface between ISG65 and C3-ANA showing residues that engage in hydrogen bonds - ISG65 K97 and ANA E689 as well as ISG65 E302 and ANA G717. B, SPR sensorgrams of interaction between ISG65 (ligand) and C3a (analyte).

### Detailed Description of the Invention

In this chapter, the term "ISG65" refers to the tested extracellular domain of ISG65 (His-ISG65₁₈₋₃₆₃).

### Cloning, expression and purification

### ISG65

A DNA fragment (Genewiz) encoding amino acids (aa) 18-363 of ISG65 from *T. b. gambiense* (Tbg.972.2.1600) was codon optimised for bacterial expression and cloned into the pET15b plasmid using Gibson assembly method (New England Biolabs). For recombinant expression in *E. coli* T7 shuffle cells (New England Biolabs), a plasmid encoding an N-terminal hexa-histidine-tag (His-ISG65₁₈₋₃₆₃) and another with an additional C-terminal Avi-tag (His-ISG65₁₈₋₃₆₃-Avi) were generated. Amino acids substitutions were introduced into the His-ISG6518-363 construct using the Q5 site-directed mutagenesis kit (New England Biolabs). For all ISG65 variants, the same expression and purification protocol was employed. Proteins were produced overnight at 22°C after induction of protein expression with 1 mM Isopropyl-b-D-thiogalacto-pyranoside (IPTG). Soluble protein was purified from bacterial lysate by immobilised metal-affinity chromatography (IMAC) using nickel-nitrilotriacetic acid (NTA) beads (Qiagen) and gravity-flow columns (Bio-Rad). After IMAC, the purified proteins were concentrated using Amicon Ultra centrifugal filters (Merck Millipore) and subjected to size-exclusion chromatography using a Superdex 200 Increase 10/300 GL (GE Healthcare) equilibrated with 20 mM HEPES (pH 7.5) and 150 mM NaCl.

The DNA sequence of the expression construct was SEQ ID NO: 9:

ATGGGCAGCAGCCATCATCATCATCATCACAGCAGCGGCCTGGTGCCGCGCGGCAGCCATATGTTGTT
AGTAATTGGCAGTGAGGATAACCGTGTTCCAGGAGACAAGAAGTTGACGAAAGAGGGAGCAGCGGCAC
TCTGTAAAATGAAGCATCTTGCTGATAAAGTTGCAAAGGAGAGATCACAAGAGTTAAAGGATAGAACT
CAAAATTTTGCTGGTTATATAGAGTTCGAGTTGTATAGAATAGATTATTGGTTGGAAAAGCTGAACGG
TCCGAAGGGGCGAAAAGACGGTTATGCTAAGCTGTCTGACTCTGATATAGAAAAAGTAAAGGAGATAT
TCAACAAGGCAAAAGATGGAATAACTAAGCAACTCCCTGAGGCAAAGAAAGCCGGTGAGGAAGCTGGA
AAACTACATACTGAAGTGAAGAAGGCCGCGGAGAATGCACGTGGACAGGATCTTGATGACGATACGGC
GAAGTCCACTGGCTTGTACAGGGTGCTGAATTGGTACTGCATTACTAAAGAAGAAAGGCACAACGCTA
CCCCTAACTGTGACGGAATCCAATTCAGAAAGCATTATCTGTCGGTGAATAGAAGCGCTATTGACTGC
AGCAGCACCAGCTATGAGGAGAATTATGATTGGTCTGCGAACGCGCTGCAGGTGGCCTTAAATAGCTG
GGAGGATGTGAAGCCAAAAAAATTGGAGTCAGCCGGGTCGGACAAGAATTGCAACATCGGCCAATCTT
CCGAGAGCCATCCATGCACCATGACAGAGGAGTGGCAGACTCCATACAAGGAAACTGTCGAAAAGCTA
AGGGAACTTGAGGATGCGTACCAAAGGGGCAAGAAGGCTCATGATGCTATGTTGGGTTACGCTAATAC
CGCATATGCTGTGAACACGAAAGTGGAACAGGAAAAGCCGCTGACAGAGGTGATAGCGGCAGCTAAGG
AAGCAGGGAAAAAGGGCGCGAAAATTATAATACCCGCAGCTGCCCCAGCAACACCGACTAACAGCACA
AAAAATGATGATAGTGCTCCAACCGAGCATGTTGATAGAGGGATTGCAACAAATGAAACACAGGTGGA
AGTTGGTATTGATTAA.

The translated sequence, including the affinity tag, was SEQ ID NO: 10:

MGSSHHHHHHSSGLVPRGSHMLLVIGSEDNRVPGDKKLTKEGAAALCKMKHLADKVAKER
SQELKDRTQNFAGYIEFELYRIDYWLEKLNGPKGRKDGYAKLSDSDIEKVKEIFNKAKDG
ITKQLPEAKKAGEEAGKLHTEVKKAAENARGQDLDDDTAKSTGLYRVLNWYCITKEERHN
ATPNCDGIQFRKHYLSVNRSAIDCSSTSYEENYDWSANALQVALNSWEDVKPKKLESAGS
DKNCNIGQSSESHPCTMTEEWQTPYKETVEKLRELEDAYQRGKKAHDAMLGYANTAYAVN

TKVEQEKPLTEVIAAAKEAGKKGAKIIIPAAAPATPTNSTKNDDSAPTEHVDRGIATNET
QVEVGID.

### ISG75 (comparative)

ISG75 was expressed and purified as previously described (doi: 10.3390/pathogens10081050 (2021)).

### Human complement factor 3

Native C3 was purified from normal human serum (Sigma-Aldrich) (as described in doi: 10.1002/0471142735.im1303s14 (2001)). Purification and generation of the fragments C3b and C3(H₂O) were carried out as described (doi: 10.1016/0022-1759(89)90340-898 2 (1989)). In short, the thioester in C3 was hydrolysed using methylamine and deactivated by incubation with iodoacetamide. C3c and C3a were purchased from Complement Technology, Inc. C3d (His-C3d) was produced recombinantly in *E*. *coli* as described (doi: 10.1021/bi0101749 (2001)). Amino acid substitutions were introduced using the same method as for ISG65. For SPR assays, a C3d construct (His-C3d-Avi) with an N-terminal His-tag and a C-terminal Avi-tag was generated. Cloning of this construct into pET15b was done by the Gibson assembly method. Expression and purification of all C3d constructs were carried out in the same way as described for ISG65.

### Binding of ISG65 to human C3

Initially, the interaction between the extracellular domain of ISG65 (His-ISG65₁₈₋₃₆₃) and human C3 was identified using IMAC-assisted pulldown assay with the extracellular domain of ISG65 (His-ISG65₁₈₋₃₆₃) and commercially obtained, non-heat inactivated human serum. 400 µL of settled Ni-NTA resin (Qiagen) were incubated with 200 µg of His-ISG65₁₈₋₃₆₃, washed with 2.5 ml IMAC wash buffer (20 mM Tris, 500 mM NaCl, 20 mM Imidazole, pH8.0) and incubated with 2.5 ml of human serum. Subsequently the beads were washed again using the same buffer, before bound protein was eluted with in a total volume of 2 ml IMAC elution buffer (20 mM Tris, 500 mM NaCl, 400 mM Imidazole, pH8.0). 4 fractions were collected and analysed by SDS-PAGE and Coomassie-staining. All steps were carried out in gravity-flow columns (Biorad). ISG65 (without serum) and serum (without ISG65 as bait protein) were used as negative controls. Specific enrichment of C3 in the presence of ISG65 was confirmed by mass spectrometry (LC-MS/MS) form cut-out gel bands.

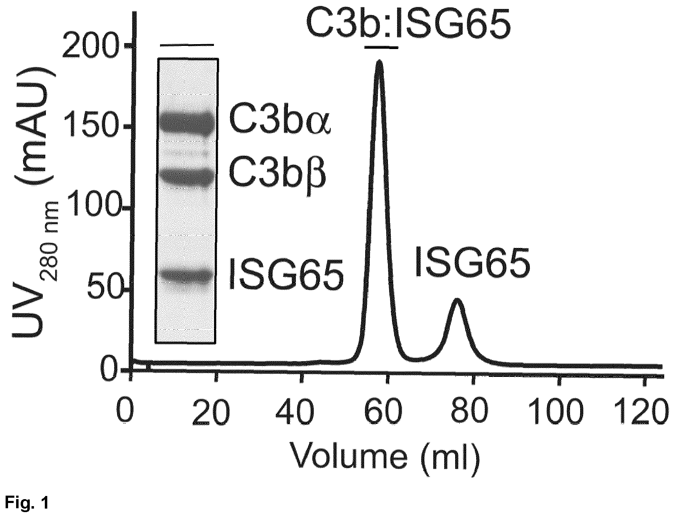

To further characterise this interaction and its stability, purified ISG65 was incubated in 3-fold molar excess with C3b and subjected to size exclusion chromatography. Two distinct peaks corresponding to the ISG65:C3b complex and excess ISG65 were observed. Analysis of peak fractions by SDS-PAGE revealed three bands that corresponded to ISG65 (39 kDa) as well as to the α- and β-chains of C3b (70, and, respectively 100 kDa), demonstrating that a stable complex was formed (Fig. 1). To further characterise the interaction, the complex was crosslinked using disuccinimidyl adipate (DSA). For cross-linking, proteins were transferred into buffer containing 20 mM HEPES (pH 7.5) and 150 mM NaCl. ISG65 and C3b were mixed in a 2:1 molar ratio, and freshly prepared DSA was added to the proteins at a hundred molar access. The reaction mixture was incubated at room temperature for 30 min and quenched with 10 molar access of ethanolamine. For EM-grid preparation, the cross-linked complex was also gel-filtered on Superdex 200 Increase 10/300 GL (GE Healthcare). SDS-PAGE analysis of the crosslinked sample showed two bands migrating at approximately 220 kDa and 39 kDa, consistent with crosslinked ISG65: C3b complex with a stochiometric ratio of 1:1 and free ISG65.

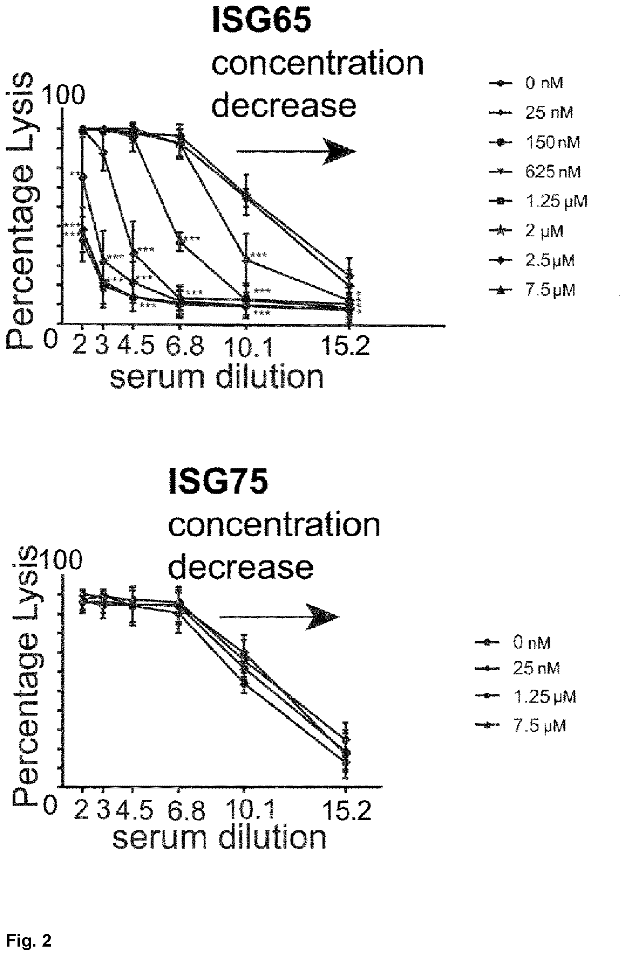

C3 and its functionally active fragment C3b are the central components of the alternative pathway (AP). To determine that IGS65 has an immune-regulatory role, a commercially available haemolytic assay (AP50 test, Haemoscan) for assessing the complement activity in human serum by quantification of complement mediated lysis of sheep erythrocytes was used according to the manufacturer's instructions. Dilutions (2-15.2-fold) of non-heat inactivated human serum (Sigma-Aldrich) were incubated with serially diluted ISG65 and ISG75 (7.5-0 µM). Complement inhibition was determined from the extent of erythrocyte lysis measured at 415 nm. The concentration of free haemoglobin is hereby directly proportional to complement activity. The measurements were performed in triplicates (n=3). Statistical significance was calculated using two-way ANOVA in GraphPad Prism. The addition of ISG65 to the human serum resulted in a concentration-dependent decrease in haemolysis, indicating an inhibition of the AP (Fig. 2). To test for the specificity of these results, the experiment was repeated using another member of the *T. brucei* invariant surface glycoprotein family, ISG75 (Fig. 2). Increasing concentrations of ISG75 had no measurable effect on haemolysis, thereby confirming an ISG65-dependent inhibition of the complement cascade *in vitro.*

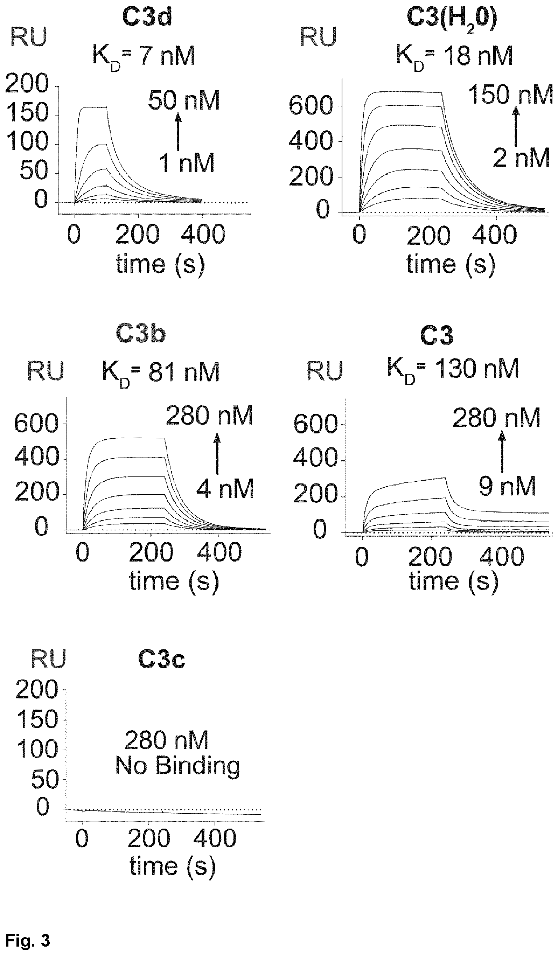

During the activation of the AP, C3 undergoes multiple proteolytic cleavages that result in an array of activated fragments, each with a specific role within the AP. To understand whether ISG65 can distinguish between these naturally occurring fragments, affinity measurements using surface plasmon resonance (SPR) were carried out (Fig. 3, Table 1). ISG65 extracellular domain was immobilised on the SPR sensor chip via its biotinylated C-terminus (His-ISG65₁₈₋₃₆₃-Avi) while the different C3 fragments were applied to the chip as analytes. This coating strategy represented a good mimetic of the presentation of native ISG65 on the cell surface via its transmembrane anchor. Binding of the two major C3 fragments, C3c and C3d, was also tested to determine the site of the ISG65 interaction. Binding impaired mutants of ISG65 and C3d were subjected to kinetic binding analysis using SPR. For interrogation of ISG65 mutants, His-C3d-Avi was used as ligand, and for C3d mutants, His-ISG65₁₈₋₃₆₃-Avi was the ligand. Site-specific Avi-tag biotinylation for immobilisation on the SPR chip was carried out as previously described (doi:10.1007/978-1-4939-2272-905 7_12 (2015)). SPR experiments were performed at 25°C on BIAcore T200 using Series S sensor chip CAP (Cytiva). All binding analyses as well as dilutions were performed in SPR running buffer (20 mM HEPES pH7.5, 150 mM NaCl, 3 mM EDTA, 0.005 % (v/v) TWEEN-20). Biotinylated ligands were coupled to flow path 2 at 10 µl.min⁻¹ for 120 s. Dilution series (1:1) of both analytes (ISG65, 1.5-100 nM; C3d, 0.78-50 nM) were applied to flow paths 1 and 2 at 30 µl.min⁻¹ for 120 s, followed by 300 s of dissociation time. The chip surface was regenerated in between titrations with 6 M guanidinium hydrochloride dissolved in 0.25 M sodium hydroxide. The binding data was reference subtracted, and kinetic and steady state affinity parameters were evaluated using BIAcore T200 evaluation software (GE 526 Healthcare). Binding analyses of other fragments were carried out in an analogous manner.

ISG65 has indeed clear preferences for certain C3 activation fragments. Native C3 (*K*D=130 nM), the most abundant C3 species in human blood, has the lowest binding affinity, followed by C3b (*K*D=81 nM) and C3(H₂O) (*K*D=18 nM). The high affinity for C3(H2O), the initiator of the AP, is likely to be a response to its low abundance in serum. The interaction with C3d (the proteolytically liberated thioester domain, TED) that takes place at even higher affinity (KD=7 nM), might be due to unimpeded movement in absence of the remaining C3 scaffold (i.e., C3c) (Fig. 3).

**Table 1: Binding affinities between ISG65 and complement factors measured by surface plasmon resonance. All data interaction parameters were calculated using the BIAcore T200 evaluation software. For each interaction, the method of K_{D} calculation is indicated. NB - no binding.**

| **Ligand** | **Analyt** | **Mutation** | ***k*ₒₙ₁ (M⁻¹s⁻¹)** | ***k*ₒₙ₂ (M⁻¹s⁻¹)** | ***k*_{off1} (s⁻¹)** | ***k*_{off2} (s⁻¹)** | ***K*_{D} (nM)** | **C3d binding (%)** |
|---|---|---|---|---|---|---|---|---|
| **Binding impaired mutants** | | | | | | | | |
| C3d-Avi | ISG65 | | | | | | | |
| | | Wt | 1003000 | 0.90 | 0.04 | 0.002 | 27.9 | 100 |
| | | Y70A | 118000 | 0.008 | 0.14 | 0.58 | 78.2 | 36 |
| | | W211A | 320000 | 0.003 | 0.16 | 0.39 | 66.8 | 42 |
| | | Y293A | - | - | - | - | NB | NB |
| | | T297A | 171000 | 0.001 | 0.05 | 0.002 | 181.8 | 15 |
| ISG65-Avi | C3d | | | | | | | **ISG65 binding (%)** |
| | | Wt | 5241000 | - | 0.03785 | - | 7.2 | 100 |
| | | L1109A | - | - | - | - | 52.9 | 45 |
| | | E1110A | - | - | - | - | 15.7 | 8 |
| | | N1134A | 1893000 | - | 0.05419 | - | 28.6 | 25 |

| **C3 fragments** | | | | | | | | |
|---|---|---|---|---|---|---|---|---|
| ISG65-Avi | C3 (H₂O) | | 941500 | - | 0.01698 | - | 18.0 | - |
| | C3b | | 262200 | - | 0.02120 | - | 80.9 | - |
| | C3 | | 81840.00 | 0.002427 | 0.05433 | 0.0005864 | 129.2 | |
| | C3c | | - | - | - | - | NB | - |
| | C3a | | - | - | - | - | 18 100 | - |
| | | | | | | | | |

### Cryo-electron microscopy (Cryo-EM) structures of ISG65 in complex with C3 and C3b:

### Grid preparation and data collection

### ISG65:C3

3 µl (0.16 mg/ml) of ISG65:C3 were applied to freshly glow-discharged copper C/Flat 1.2/1.3 300 mesh grids (Protochips) and vitrified by being plunged into liquid ethane using a ThermoFisher Scientific Vitrobot Mark IV system (4 °C, 100% relative humidity, no wait time, 2 s blotting time). The grids were then transferred to a Titan Krios G3i microscope (ThermoFisher Scientific) equipped with a K3 detector (GATAN Inc.) and operated at 300 kV. Images were recorded at a magnification of 105000X while tilting the stage by 25°, yielding a pixel size of 0.86Å. Multi-frame movies (40 frames with total dose of 41.10 e⁻/Å²) were recorded using a nominal defocus (dF) range of - 2.5 to - 1.5 µm. Data were collected using the automated data collection software EPU (ThermoFisher Scientific).

### ISG65:C3b

To prepare grids used in the collection of datasets at a 25° tilt, 3.5 µl (0.1 mg/ml) of ISG65:C3b complex was applied to glow-discharged copper C/Flat, 300 mesh 1.2/1.3, and 2/2 TEM grids (Protochips). The grids were vitrified by being plunged into liquid ethane using ThermoScientific Vitrobot Mark IV system (4°C, 100% rel. humidity, 30 s waiting time, 4 s blotting time) and then transferred to a Titan Krios microscope (ThermoFisher Scientific) for data acquisition.

For data collections at a 0° stage tilt, Au, 300 mesh, R1.2/1.3 TEM grids (Protochips) were coated with a graphene monolayer using an in-house developed protocol. 3.5 µl (0.15 mg/ml) of ISG65:C3b complex was applied to freshly plasma-cleaned TEM grids and vitrified as explained for grids used to obtain the 25° tilt dataset. The grids were subsequently transferred to a Titan Krios microscope (ThermoFisher Scientific) for data acquisition.

The data were collected at 300 kV using SerialEM44 software. The data were collected on a K2 direct electron detection camera positioned behind a Gatan Imaging Filter (Bioquantum 967, Gatan). The camera was operated in the electron counting mode, and the data were collected at the calibrated pixel size of 0.818 Å/px. The data from a 5.0 s exposure were split into 40 frames comprising an overall dose of 60 e⁻/Å².

### Image processing

### ISG65:C3

A total of 10,380 movies were acquired during data collection. Motion correction as well as CTF correction (CTFFIND4 wrapper) were done using cryoSPARC⁴⁵. 5,508,854 particles were picked using the reference-free Gaussian Blob picker in cryoSPARC. Particles were extracted with a 400 px box with 2- fold binning applied which resulted in a pixel size of 1.72 Å/px. Iterative panning using reference free 2D classification in cryoSPARC was performed until a subset of 432,224 particles was identified. The particles were re-extracted using a 400 px box with no binning applied. The re-extracted particles were submitted to another round of reference free 2D classification in Relion 3.1⁴⁶. 406,545 particles were selected and used for *ab initio* modelling and subsequently subjected to the 3D classification job in Relion. Particles were classified into 5 classes, all yielding well defined reconstructions of the desired complex. All particles selected after 2D classification in Relion were later used for creation of an *ab initio* model in cryoSPARC, which was subsequently refined using the non-uniform refinement procedure (cryoSPARC v3.2 and later) and local CTF refinement to yield the final reconstruction.

### ISG65:C3b

A total of 18,062 movies were collected at a 25° stage tilt in two separate data collections (for two different grid types), and 8960 movies were collected at a 0° stage tilt on graphene coated grids. The processing of each dataset was performed independently before merging selected particles with previous data collections.

Motion correction and CTF correction for all 27,022 movies were performed using cryoSPARC's patch motion and patch CTF correction implementations, respectively.

On images acquired at a 25° stage tilt, particles were picked using the convolutional neural network-based particle picker, crYOLO⁴⁷. Particles were initially identified using the supplied general model. The resulting particle picks were manually inspected, and reference free 2D classification was performed in cryoSPARC. Particles constituting the 2D classes which represented the protein (regardless of whether they were the desired complex or not) were selected and used to re-train the outer layers of the general model on a subset of micrographs at varying defocus. crYOLO identified a total of 2,836,445 particles (1,414,868 and 1,421,577).

The processing of the two datasets followed the same overall processing pipeline, using cryoSPARC. Particles were extracted using a box with 400 px and two-fold binning, resulting in a pixel size of 1.656 Å/px.

Iterative, reference-free 2D classification was performed until satisfactory 2D classes were identified. The selected particles were subjected to *ab initio* modelling using 3 classes. Models representative of the desired ISG65:C3b complex and C3b were chosen for subsequent Heterogenous Refinement. Particles constituting the reconstruction representing ISG65:C3b were selected, re-extracted using a 400 px box (no binning applied resulted in a pixel size of 0.828 Å/px), and subjected to Non-uniform Refinement.

For images acquired at a 0° stage tilt, the gaussian particle picker in cryoSPARC was utilised to identify particles. The remaining processing was performed as described for data collected at a 25° stage tilt.

For the final reconstruction, 204,946 combined particles were re-subjected to *ab initio* modelling using 2 classes. The class representative of the desired complex was subjected to Non-uniform Refinement and local CTF refinement.

A total of 145,172 particles, of which 54,453 particles came from datasets at 25° tilt (22,266 and 32,187, respectively) and 90,719 particles came from a 0° tilt dataset, contributed to the final reconstruction.

### Model building and refinement

### ISG65: C3

The initial step in model building was performed by docking starting models (the crystal structure of C3 [PDB 2A73] and an ISG65 model predicted by AlphaFold2⁴⁸) into the obtained electron density map using Phenix⁴⁹. After an initial real space refinement in Phenix, both models were independently and iteratively refined using a combination of manual refinements in Coot as well as automated real-space refinements using Phenix and REFMAC5⁵⁰. Model validation was performed using MolProbity⁵¹.

After the models had individually reached satisfactory validation metrics and map correlation, they were manually and automatically refined together using Coot⁵² and Phenix⁴⁹ to model interactions between the two proteins.

### ISG65: C3b

Due to large variations in local resolution of the obtained electron density map, the modelling of ISG65 at atomic resolution was not possible. Therefore, the atomic model obtained from the ISG65:C3 data was simply docked in the electron density map using Phenix. For modelling C3b, a starting model (the crystal structure of C3b [PDB 2107]) was docked in the obtained electron density via Phenix. After an initial real space refinement in Phenix, the model was iteratively refined using a combination of manual refinements in Coot as well as automated real-space refinements using Phenix and REFMAC5. Model validation was performed using MolProbity.

### Structure description

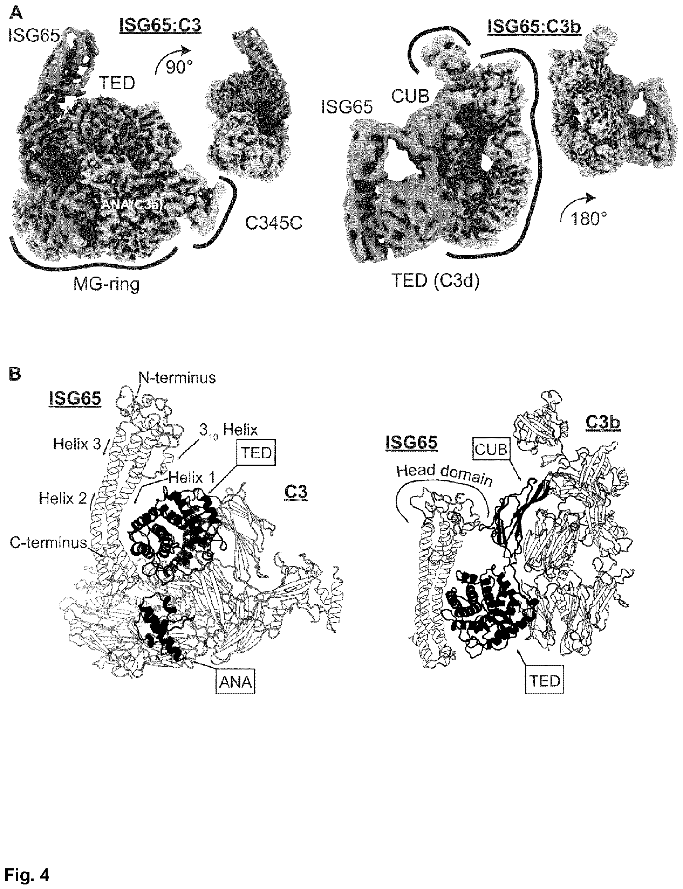

The ISG65:C3 complex could be reconstructed to a reported FSC0.143 of 3.58 Å, while ISG65:C3b was reconstructed to FSC 0.143 of 3.59 Å. However, the local resolution range across the reconstructions varies significantly, especially for ISG65:C3b. The resolution of the reconstruction corresponding to the rather rigid MG-ring is in the near-atomic range of 3.5 Å. The local resolution in the CUB and TED region varies between 4 and 5.5 Å, while at the interface and in the remainder of ISG65, it ranges from 5 to beyond 10 Å. Secondary structure features like β-sheets in the CUB domain and α-helices in ISG65, however, could still be identified (Fig. 4A). For ISG65:C3, the local resolution predominantly ranges from 3 to 5 Å, with lower resolutions in the disordered head region of ISG65, the flexible C345c domain, and the glycan trees in the two glycosylation sites. The higher variance in local resolution across the ISG65:C3b reconstruction is likely caused by an increased flexibility of the extended domains CUB and TED relative to the MG-ring. In contrast, C3 adopts a more compact conformation with TED wedged between the MG-ring and ANA, resulting in reduced flexibility and thus variance within the molecule. This is also reflected in the sloped shape of the FSC of ISG65:C3b, and a rather vertical drop of the FSC in the ISG65:C3 reconstruction.

### ISG65:C3b

Despite large variations in the local resolution across the reconstruction, C3b could be modelled except for the flexible regions Asn93-Gly101, Gln312-Leu314, Pro665-Ala667, Ser749-Leu751, Glu1372-Ala1380, and Ser1523-Asp1524. C3b resembles previously published structures (e.g., PDB 2107). Minor re-arrangements of the TED and the CUB domain, especially in residues Gln1161-Pro1287, can be observed in C3b and may be caused by ISG65 binding (data not shown).

The lack of near-atomic resolution in ISG65 of the ISG65:C3b reconstruction prevented us from drawing conclusions about details of the binding interface. However, the superposition of the atomic model of ISG65 with its counterpart in the C3 complex suggests an identical binding interface with TED. In addition to the TED interface, the electron density corresponding to the N-terminal 'head' region of ISG65 also suggests contacts to the CUB domain on C3b (Fig. 4A, right). Should this interaction occur in a physiological context, the second attachment point could lock the TED domain in a conformation that further restricts its free movement. This lock would thereby also prevent the movement of the hydrolysed thioester bond of Gln1013 towards the membrane.

### ISG65:C3

The near atomic resolution of the ISG65:C3 reconstruction allowed us to build the first reported model of a *T*. *b. gambiense* Invariant Surface Glycoprotein and to describe its interaction with human complement C3 on a structural basis.

ISG65 adopts a three-helix bundle fold (Helix 1, Lys36-Lys84; Helix 2, Asp100-198 Asn144; Helix 4, Thr259-Glu300) with a 20° curvature along its longest axis . The disordered N-terminal residues Leu18-Leu34 as well as 200 residues Arg146-Ser202, and Lys227-Met255 constitute intrinsically disordered 201 loops and thus were not resolved. Instead of distinct conformations, a cloud of 202 electron density was observed, describing the average volume occupied by the loops. Using MS-based disulphide mapping, we were able to identify three disulphide bridges in the disordered 'head' region of ISG65 (Cys12-Cys137, Cys149-Cys168, and Cys209-Cys220). The atomic model of the disordered regions was predicted using iterative, template-guided modelling in AlphaFold2 using real space constraints from the reconstruction and disulphide bonding as selection criteria for the models. Although resolved at a lower local resolution, the loop connecting helix 1 and helix 2 (Lys85-Asp100) close to the C-terminus could be modelled. Due to a well resolved electron density, we could confidently place the short helix 3 (residues Gln217 to Val226) and the preceding loop (Asp210-Leu216) near the 'head' region of ISG65. Residues Thr203-Asp210 were not resolved to atomic resolution but appear to have a 310 helical conformation. At the C-terminal end of the model, residues Gln301-Leu305 form a loop connecting helix 4 with the short helix 5 (Thr306-Ala312), which notably points towards the N-terminus of the protein, away from the parasite surface. Beyond these residues, only poorly resolved electron density could be observed which made accurate placement of residues in this region impossible. With the exception of the disordered regions Ala666-Ser672, Arg740-Leu751, and Glu1634-Asn1642, C3 was well resolved and could be modelled in its entirety (Fig. 2B). Positioned on top of the ANA domain (C3a, when released), binding of ISG65 to C3 is predominantly mediated via multiple hydrophobic contacts and four distinct hydrogen bonds with the thioester domain (Arg77_{ISG65}- Leu1109_{C3}) and three hydrogen bonds from Tyr293 (Tyr293_{ISG65}-Glu1110_{C3}, Tyr293_{ISG65}-Glu1111_{C3} and Tyr293_{ISG65}-Glu1119_{C3}). The interaction between the proteins is further facilitated by the almost perfect complementary fit between the convex tip of TED and the concave 3-helix-bundle of ISG65, a likely result of molecular co-evolution (Fig. 2).

### Validation of the interface

In order to dissect the binding interface into segments and key residues critically contributing to the overall binding affinity, we mutated surface-exposed, non-structurally relevant residues to alanines. These key residues were chosen at the upper, middle, and lower end of the binding site in ISG65 as well as at the convex tip of TED, based on the higher resolution structure of ISG65: C3. The binding affinities of recombinantly expressed C3d mutants to ISG65, and vice versa, ISG65 mutants to C3d (representing TED in C3), were determined by SPR and compared against the affinities of the wild-type (wt) proteins. Correct protein folding of the mutants was assessed via circular dichroism spectroscopy (CDS). ISG65 mutations along the entire length of the interface contributed to the binding as indicated by the decrease in dissociation constants (*K_{D}*s) (Table 1). Mutation of Tyr293 completely abrogated ISG65 binding to C3d. As Tyr293 is one of the residues that contributes hydrogen bonds to the interaction with C3d and engages in several hydrophobic contacts with C3d residues as well, a severe reduction in binding affinity was to be expected. Notably, mutation of Trp211 caused a decrease in binding affinity to 42%. This is in agreement with the well-resolved electron density of the adjacent loop (Asp210-Leu216) as well as the side chain of Trp211. Although only contributing two hydrophobic contacts, this interaction might be crucial for correct alignment of C3d at the upper end of the interface. Similarly, mutation of two of the four hydrogen bond forming residues on C3d, Leu1109 and Glu1011 leads to a notable decrease in binding affinity. This can also be attributed to the fact that the side chains of Leu1109 and Glu1110 are located at the core of the interface and engage in multiple hydrophobic contacts. Despite its localisation outside the interface in the ISG65:C3 structure, the mutation of Arg1134 on C3d caused a decrease in binding affinity to 24%. To aid the identification of potential key mediators of the interaction, we also employed hydrogen-deuterium exchange mass spectrometry (HDX-MS). Here, Arg1134 was centred in a 'protection hotspot', indicating limited solvent accessibility. When taken into consideration that this residue is located on a long (26 aa) loop close to the interface, it seems conceivable that a transient interaction with ISG65 binding may occur.

### ISG65 interacts with ANA/C3a

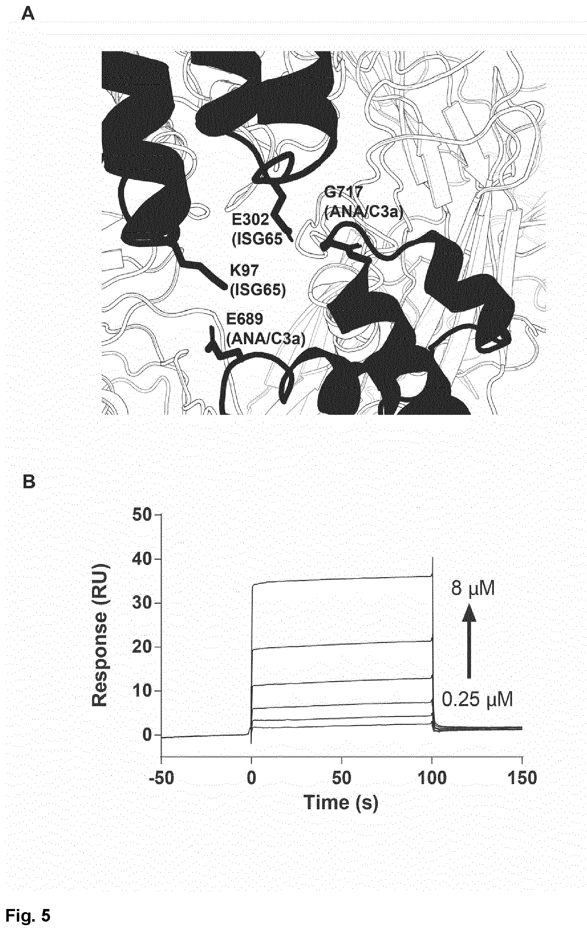

In the ISG65:C3 structure, a second, smaller interface is formed between the membrane proximal, C-terminal end of ISG65 and the ANA domain. Within the interface, we could identify two possible hydrogen bonds, Lys97_{ISG65} - Glu689_{C3} and Glu302_{ISG65} - Gly717_{C3} (Fig. 5A). Although the reconstruction in this region suffers from poor electron density for the side chains, the identified residues are well within bonding distance (Supplementary Table 2). Furthermore, all contributing residues are located in loops of varying lengths and thus are potentially subject to large degrees of freedom, facilitating transient interactions. To investigate whether the interface between ANA and ISG65 may be physiologically relevant, we confirmed the specific binding of recombinant C3a to ISG65 by SPR (Fig. 5B, Table 1). In agreement with the lack of clear electron density, kinetic profiles revealed high on- and off-rates which is indeed indicative of a rather short-lived interaction between ISG65 and free C3a.

## Claims

1. ISG65-derived protein having the length of up to 500 amino acid residues and containing an amino acid sequence having at least 90% identity, preferably at least 95% identity, to the sequence SEQ ID NO: 8, wherein the underlined regions are conserved: 
GYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QE (SEQ ID NO: 8)
 for use as a medicament.

2. ISG65-derived protein according to claim 1 for use in the treatment of a complement dysregulation disease.

3. ISG65-derived protein according to claim 1 for use in the treatment of a disease selected from atypical hemolytic uremic syndrome (aHUS), C3 glomerulopathy (C3G), paroxysmal nocturnal hemoglobinuria (PNH), autoimmune diseases, Alzheimer's syndrome, schizophrenia, angioedema, macular degeneration, and Crohn's disease.

4. ISG65-derived protein for use according to any one of claims 1-3, wherein the ISG65-derived protein has the length of up to 500 amino acid residues and contains an amino acid sequence having at least 90% identity, preferably at least 95% identity, to the sequence SEQ ID NO: 5, wherein the underlined regions are conserved:

LLVIGSEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEVIAAAKEAGKKGAKIIIPAAAPATPTNSTKNDDSAPTEHVDRGIATNETQVEV
GID (SEQ ID NO: 5).

5. ISG65-derived protein for use according to any one of claims 1-3, wherein the ISG65-derived protein has the length of up to 500 amino acid residues and contains an amino acid sequence having at least 90% identity, preferably at least 95% identity, to the sequence SEQ ID NO: 6, wherein the underlined regions are conserved:

MMKYLLVFAIIATRIPVLLVIGSEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEVIAAAKEAGKKGAKIIIPAAAPATPTNSTKNDDSAPTEHVDRGIATNETQVEV
GIDAD (SEQ ID NO. 6).

6. ISG65-derived protein for use according to any one of claims 1-3, wherein the ISG65-derived protein has the length of up to 500 amino acid residues and contains an amino acid sequence having at least 90% identity, preferably at least 95% identity, to the sequence SEQ ID NO: 7, wherein the underlined regions are conserved:

SEDNRVPGDKKLTKEGAAALCKMKHLADKVAKERSQEL
KDRTQNFAGYIEFELYRIDYWLEKLNGPKGRKDGYAKLSDSDIEKVKEIFNKAKDGITKQ
LPEAKKAGEEAGKLHTEVKKAAENARGQDLDDDTAKSTGLYRVLNWYCITKEERHNATPN
CDGIQFRKHYLSVNRSAIDCSSTSYEENYDWSANALQVALNSWEDVKPKKLESAGSDKNC
NIGQSSESHPCTMTEEWQTPYKETVEKLRELEDAYQRGKKAHDAMLGYANTAYAVNTKVE
QEKPLTEV (SEQ ID NO: 7).

7. A pharmaceutical formulation containing the ISG65-derived protein as described in any one of the claims 1, 4-6 and at least one pharmaceutically acceptable excipient.

8. The pharmaceutical formulation according to claim 7 wherein the pharmaceutically acceptable excipient is selected from water, buffer, stabilizer, surfactant, binder, filler and glidant.
